# EUROPEAN PATENT APPLICATION

(11) **EP 4 300 081 A1**
(43) Date of publication of application: **03.01.2024**
(21) Application number: 22758716.9
(22) Date of filing: 19.01.2022
(51) Int. Cl.: G01N 21/76, G01N 33/50, G01N 33/53, G01N 35/00

(54) **SAMPLE JOINT INSPECTION AND ANALYSIS SYSTEM**

(30) Priority: 24.02.2021 CN 202110205576
(71) Applicant: Shenzhen Yhlo Biotech Co., Ltd., 518116 Shenzhen (CN)
(72) Inventor: ZHANG, Fuxing, Shenzhen, Guangdong 518116 (CN); XIAO, Yujin, Shenzhen, Guangdong 518116 (CN); HU, Kunhui, Shenzhen, Guangdong 518116 (CN)
(74) Representative: Michalski Hüttermann & Partner Patentanwälte mbB
(86) International application number: PCT/CN2022/072681
(87) International publication number: WO 2022/179350

(57) **Abstract**

A sample joint inspection and analysis system (10), comprising a rack, and a sample processing apparatus (100), a chemiluminescence immunoassay apparatus (200) and a biochemical analysis apparatus (300) which are arranged on the rack, wherein the sample processing apparatus (100) comprises an immune sample injection channel (110) and a biochemical sample injection channel (120), the immune sample injection channel (110) being in communication with the chemiluminescence immunoassay apparatus (200), and the biochemical sample injection channel (120) being in communication with the biochemical analysis apparatus (300). The sample joint inspection and analysis system (10) has a miniature structure and performs inspection rapidly; multi-item joint inspection based on a biochemical reaction principle and a chemiluminescence inspection principle can be realized; the test advantages of a biochemical reaction and an immune reaction are exploited, and the two test modes do not interfere with each other; the requirements of various medical and inspection institutions or departments for high-throughput and high-sensitivity multiple joint inspection can be met; and multiple options are also provided for a patient.

## Description

### TECHNICAL FIELD

The present disclosure relates to a field of medical device and biological detection, in particular to a sample joint inspection and analysis system.

### BACKGROUND

In the field of in vitro diagnosis with a medical test equipment, the trend of joint multi-item index detection, large-scale detection and packaged detection is becoming increasingly evident. For example, C-reactive protein (CRP), serum amyloid A (SAA) and procalcitonin (PCT) are the main basis for experimental examination of infectious diseases, which are used for diagnosis and differentiation of infections. However, the requirements for test sensitivity and linearity range of different items are different due to the varying content of different analytes in the sample. Meanwhile, due to the limitation of the technical condition of detection, it is difficult to achieve joint detection for multiple antigens or antibody with large content difference (the content difference is more than 100 times) in a single sample at present. As for the detection of the CRP, SAA and PCT described above, CRP and SAA are substances at the mg level and PCT is a substance at the pg level. In this case, items such as CRP and SAA, which can be detected by immunoturbidimetry based on biochemical reaction principle, are still detected by immunoturbidimetry. For the highly sensitive PCT item, chemiluminescent detection is performed. The two detection methods are based on completely different testing systems. In conventional detection systems, different testing systems are distributed in different instruments, so a plurality of tubes of samples need to be taken and distributed to different instruments, or a tube of sample is moved to another instrument for testing after being tested on one instrument, which will bring great inconvenience to customers and detection staff. In addition, due to the limitations of financial resources, material resources and site space, many grass-roots health units cannot install a plurality of different kinds of detection instruments at the same time to configure a plurality of different testing systems. However, there is a need for a plurality of different test systems to detect the patients at the same time. Therefore, the existing detection conditions of the grass-roots health units are difficult to meet the detection requirements of the patients.

### SUMMARY

Based on the above, it is necessary to provide a sample joint inspection and analysis system. The sample joint inspection and analysis system of the present disclosure is miniaturized in structure and rapid in detection, which can realize multi-item joint detection based on biochemical reaction principle and chemiluminescent detection principle, and take the testing advantages of both the biochemical and immune reactions. The two test modes will not interfere with each other, which can meet the needs of multi-item joint detection with high throughput and high sensitivity in various medical and testing institutions or units. At the same time, it also provides multiple options for patients.

A sample joint inspection and analysis system includes a machine frame; and a sample processing device, a chemiluminescent immunoassay device, and a biochemical assay device that are provided on the machine frame. The sample processing device includes an immune sample entering channel and a biochemical sample entering channel. The immune sample entering channel is in communication with the chemiluminescent immunoassay device. The biochemical sample entering channel is in communication with the biochemical assay device.

In one of the embodiments, the sample processing device further includes a sample entering bin mechanism. The sample entering bin mechanism includes a sample entering seat, a sample entering rack, a first sample entering driving member, and a second sample entering driving member. The sample entering seat is mounted to the machine frame. The sample entering rack is movably connected to the sample entering seat. The sample entering rack is provided with a plurality of sample stations configured to place sample racks in parallel. The first sample entering driving member is connected to the sample entering rack to drive the sample entering rack to move along a first direction. Each of the sample stations is configured to be aligned with the immune sample entering channel and the biochemical sample entering channel respectively. The second sample entering driving member is mounted to the sample entering seat to apply an action to the sample rack in each sample station, so as to move the sample rack outwards to the immune sample entering channel or the biochemical sample entering channel along a second direction.

In one of the embodiments, the immune sample entering channel is parallel to the biochemical sample entering channel. The first direction is perpendicular to the immune sample entering channel or the biochemical sample entering channel. The second direction is parallel to the immune sample entering channel or the biochemical sample entering channel.

In one of the embodiments, the chemiluminescent immunoassay device includes a reaction cup arranging mechanism, a reaction cup feeding mechanism, a vortex mixer, an immune incubator, a reaction cup moving mechanism, a reading station, an immune magnetic separation cleaning mechanism, an immune reagent disk, and an immune feeding mechanism. The reaction cup arranging mechanism is configured to arrange and order reaction cups. The reaction cup feeding mechanism is connected to the reaction cup arranging mechanism. The reaction cup feeding mechanism has a plurality of reaction cup stations configured to receive the reaction cups. The reaction cup moving mechanism is configured to transfer the reaction cup between the reaction cup feeding mechanism, the vortex mixer, the immune incubator, the reading station, and the immune magnetic separation cleaning mechanism. The immune feeding mechanism is configured to obtain an immune reagent and a sample from the immune reagent disk and the immune sample entering channel respectively, and add the immune reagent and the sample into the reaction cup in the immune incubator.

In one of the embodiments, the immune feeding mechanism includes an immune sample feeding syringe and an immune syringe cleaning tank. Both the immune sample feeding syringe and the immune syringe cleaning tank are provided on the machine frame. The immune sample feeding syringe is capable of obtaining the immune reagent and the sample from the immune reagent disk and the immune sample entering channel respectively, and adding the immune reagent and the sample into the reaction cup in the immune incubator. The immune syringe cleaning tank is configured to clean the immune sample feeding syringe.

In one of the embodiments, the immune feeding mechanism further includes an immune sample feeding arm, a first immune sample feeding driving member, and a second immune sample feeding driving member. The immune sample feeding syringe is connected to the immune sample feeding arm. The first immune sample feeding driving member is mounted to the machine frame and connected to the immune sample feeding arm. The first immune sample feeding driving member is configured to drive the immune sample feeding arm to rotate horizontally, so as to transfer the immune sample feeding syringe between the immune reagent disk, the immune sample entering channel, the immune incubator, and the immune syringe cleaning tank.

In one of the embodiments, the reaction cup arranging mechanism, the reaction cup feeding mechanism, the vortex mixer, the immune incubator, the reading station, and the immune magnetic separation cleaning mechanism are sequentially distributed.

In one of the embodiments, the reaction cup moving mechanism includes a first linear guiding member, a second linear guiding member, a reaction cup moving seat, a reaction cup gripper, a first moving driving member, and a second moving driving member. The first linear guiding member extends from the reaction cup feeding mechanism to the immune magnetic separation cleaning mechanism. The reaction cup moving seat is slidably connected to the first linear guiding member. The first moving driving member is connected to the reaction cup moving seat to drive the reaction cup moving seat to move along the first linear guiding member. The second linear guiding member is connected to the reaction cup moving seat and extends along a vertical direction. The reaction cup gripper is slidably connected to the second linear guiding member. The second moving driving member is mounted to the reaction cup moving seat and connected to the reaction cup gripper. The second moving driving member is configured to drive the reaction cup gripper to move along the second linear guiding member.

In one of the embodiments, the reaction cup arranging mechanism includes a bin, a cup collecting assembly, and an ordering assembly, the cup collecting assembly includes cup collecting seats, a cup collecting chain, and a cup collecting driving member. A portion of the cup collecting chain extends into the bin and another portion thereof extends out of the bin. A plurality of the cup collecting seats are sequentially connected to the cup collecting chain. The cup collecting seat is provided with a cup collecting groove configured to hold one reaction cup. The cup collecting driving member is connected to the cup collecting chain to drive the cup collecting chain to move. The ordering assembly includes an ordering track, a transfer pushing member, and a reaction cup transfer driving member. The ordering track is provided with a chute configured to receive the reaction cup. A height difference is formed between a feeding end and a discharging end of the chute to enable the reaction cup to slide out. The discharging end of the chute extends to the reaction cup station of the reaction cup feeding mechanism. The transfer pushing member is configured to push the reaction cup in the cup collecting groove into the feeding end of the chute.

In one of the embodiments, the reaction cup feeding mechanism includes a feeding turntable and a rotating driving member. The feeding turntable is provided with a plurality of reaction cup stations distributed at equal intervals along a circumferential direction of the feeding turntable. The rotating driving member is connected to the feeding turntable to drive the feeding turntable to rotate.

In one of the embodiments, the biochemical assay device includes a biochemical feeding mechanism, a biochemical reagent disk, a cleaning mechanism, a biochemical incubator, a stirring mechanism, a detection light source, an optical detection module, and an ISE ion detection module. The biochemical feeding mechanism is mounted to the machine frame to obtain a biochemical reagent and a sample from the biochemical reagent disk and the biochemical sample entering channel respectively, and add the biochemical reagent and the sample to a reaction vessel in the biochemical incubator. The stirring mechanism is connected to the machine frame to stir the sample in the reaction vessel. The detection light source and the optical detection module are arranged oppositely inside and outside the biochemical incubator to detect an optical signal of the reaction vessel. The ISE ion detection module is provided on the machine frame to detect the concentration of one or more ion in the sample. The cleaning mechanism is configured to clean the reaction vessel in the biochemical incubator.

In one of the embodiments, the biochemical feeding mechanism includes a biochemical cleaning tank, a biochemical sample feeding syringe, a biochemical reagent syringe, and a reagent syringe cleaning tank that are provided on the machine frame. The biochemical sample feeding syringe is capable of obtaining the sample from the biochemical sample entering channel and adding the sample to the reaction vessel in the biochemical incubator. The biochemical syringe cleaning tank is configured to clean the biochemical sample feeding syringe. The biochemical reagent syringe is capable of obtaining the biochemical reagent from the biochemical reagent disk and adding the biochemical reagent to the reaction vessel in the biochemical incubator. The reagent syringe cleaning tank is configured to clean the biochemical reagent syringe.

In one of the embodiments, the biochemical feeding mechanism further includes a biochemical sample feeding arm, a first biochemical sample feeding driving member, and a second biochemical sample feeding driving member. The biochemical sample feeding syringe is connected to the biochemical sample feeding arm. The first biochemical sample feeding driving member is mounted to the machine frame and connected to the biochemical sample feeding arm. The first biochemical sample feeding driving member is configured to drive the biochemical sample feeding arm to rotate horizontally, so as to transfer the biochemical sample feeding syringe between the immune sample entering channel, the biochemical incubator, and the biochemical cleaning tank.

Additionally or alternatively, the biochemical feeding mechanism further includes a reagent feeding arm, a first reagent feeding driving member, and a second reagent feeding driving member. The biochemical reagent syringe is connected to the reagent feeding arm. The first reagent feeding driving member is mounted to the machine frame and connected to the reagent feeding arm. The first reagent feeding driving member is configured to drive the reagent feeding arm to rotate horizontally, so as to transfer the biochemical reagent syringe between the biochemical reagent disk, the biochemical incubator, and the reagent syringe cleaning tank.

The above-mentioned sample joint inspection and analysis system is miniaturized in structure and rapid in detection, which can realize multi-item joint detection based on biochemical reaction principle and chemiluminescent detection principle, and take the testing advantages of both the biochemical and immune reactions. The two test modes will not interfere with each other, which can meet the needs of multi-item joint detection with high throughput and high sensitivity in various medical and testing institutions or units. At the same time, it also provides patients with multiple options. The biochemical assay and immunoassay of the above-mentioned sample joint inspection and analysis system are performed simultaneously, realizing high-throughput biochemical-immune multiple-item joint detection.

In summary, the above sample joint inspection and analysis system has the following advantages:
(1) The sample processing device provides the immune sample entering channel and the biochemical sample entering channel, the biochemical sample entering channel provides the sample for the biochemical assay device, and the immune sample entering channel provides the sample for the chemiluminescent immunoassay device.
(2) The biochemical assay device retains the biochemical cleaning tank, the biochemical sample feeding syringe, the biochemical reagent syringe, the reagent syringe cleaning tank and the stirring mechanism required for biochemical reaction, and retains the characteristics of short reaction time and fast throughput in biochemical reaction.
(3) The chemiluminescent immunoassay device retains the reaction cup arranging mechanism, the reaction cup feeding mechanism, the vortex mixer, the immune incubator, the reaction cup moving mechanism, the reading station, the immune magnetic separation cleaning mechanism, the immune reagent disk and the immune feeding mechanism required for chemiluminescent reaction, and retains the syringe cleaning, reaction magnetic cleaning and dark chamber photometry requirements required for high sensitivity of chemiluminescent reaction.
(4) The sample joint inspection and analysis system enables the collection of only one tube of blood from a patient, a single operation interface, and a unified presentation of results.

### BRIEF DESCRIPTION OF THE DRAWINGS

FIG. 1 is a schematic view of a sample joint inspection and analysis system according to an embodiment of the present disclosure.
FIG. 2 is a schematic view of a sample processing device in the sample joint inspection and analysis system shown in FIG. 1.
FIG. 3 is a schematic view of an immune feeding mechanism in the sample joint inspection and analysis system shown in FIG. 1.
FIG. 4 is a schematic view of a reaction cup transfer mechanism of the sample joint inspection and analysis system shown in FIG. 1.
FIG. 5 is a schematic view of a reaction cup arranging mechanism of the sample joint inspection and analysis system shown in FIG. 1.
FIG. 6 is a schematic view of a first feeding mechanism of the sample joint inspection and analysis system shown in FIG. 1.
FIG. 7 is a schematic view of a second feeding mechanism of the sample joint inspection and analysis system shown in FIG. 1.

### Description of reference signs:

10, sample joint inspection and analysis system; 100, sample processing device; 110, immune sample entering channel; 120, biochemical sample entering channel; 130, sample entering bin mechanism; 131, sample entering seat; 132, sample entering rack; 1321, sample station; 133, first sample entering driving member; 134, second sample entering driving member; 200, chemiluminescent immunoassay device; 210, reaction cup arranging mechanism; 211, bin; 212, cup collecting assembly; 2121, cup collecting seat; 21211, cup collecting groove; 2122, cup collecting chain; 2123, cup collecting driving member; 213, ordering assembly; 2131, ordering track; 21311, chute; 2132, transfer pushing member; 2133, reaction cup transfer driving member; 220, reaction cup feeding mechanism; 221, feeding turntable; 2211, reaction cup station; 222, rotating driving member; 230, vortex mixer; 240, immune incubator; 250, reaction cup moving mechanism; 251, first linear guiding member; 252, second linear guiding member; 253, reaction cup moving seat; 254, reaction cup gripper; 255, first moving driving member; 256, second moving driving member; 260, reading station; 270, immune magnetic separation cleaning mechanism; 280, immune reagent disk; 290, immune feeding mechanism; 291, immune sample feeding syringe; 292, immune syringe cleaning tank; 293, immune sample feeding arm; 294, first immune sample feeding driving member; 295, second immune sample feeding driving member; 300, biochemical assay device; 311, first feeding mechanism; 3111, biochemical sample feeding syringe; 3112, biochemical cleaning tank; 3113, biochemical sample feeding arm; 3114, first biochemical sample feeding driving member; 3115, second biochemical sample feeding driving member; 312, second feeding mechanism; 3121, biochemical reagent syringe; 3122, reagent syringe cleaning tank; 3123, reagent feeding arm; 3124, first reagent feeding driving member; 3125, second reagent feeding driving member; 320, biochemical reagent disk; 330, cleaning mechanism; 340, biochemical incubator; 350, stirring mechanism; 360, detection light source; 370, optical detection module; 380, ISE ion detection module; 400, machine frame; 20, sample rack; 21, blood collection tube; 30, reaction cup; and 40, reaction vessel.

### DETAILED DESCRIPTION OF THE EMBODIMENTS

In order to make the objectives, features, and advantages of the present disclosure more apparent and understandable, the specific implementations of the present disclosure will be explained in detail below in conjunction with the accompanying drawings. In the following description, numerous specific details are set forth in order to facilitate a thorough understanding of the present disclosure. However, the present disclosure can be implemented in many other ways different from those described herein, and those skilled in the art can make similar modifications without departing from the spirit of the disclosure, so that the present disclosure is not limited to the specific embodiments disclosed below.

In the description of the present disclosure, it is to be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. indicate the orientations or positional relationships on the basis of the drawings. These terms are only intended for facilitating illustrating the present disclosure and simplifying the illustration, rather than indicating or implying that the devices or elements referred thereto have to present particular orientations, and be constructed and operated in particular orientations, and therefore cannot be construed as limiting the present disclosure.

In addition, the terms "first" and "second" are only intended for illustrative purposes, rather than being construed as indicating or implying relative importance or implicitly designating the number of the technical features as indicated. Thus, the features modified by "first" and "second" may explicitly or implicitly include at least one said feature. In the illustrations of the present disclosure, the term "a plurality of" means at least two, for example two or three, unless otherwise explicitly and specifically defined.

In the present disclosure, unless otherwise expressly specified and defined, the terms "mounted", "connected to", "coupled" and "fixed" should be understood in a broad sense, for example, fixedly connected or detachably connected, or integrated; mechanically connected or electrically connected; directly connected or indirectly connected through an intermediate medium, or in an interior communication or mutual interaction relationship between two elements, unless otherwise specifically defined. For those of ordinary skill in the art, the specific meanings of the above-described terms in the present disclosure may be understood according to specific circumstances.

In the present disclosure, unless otherwise expressly stated and defined, the first feature, when being referred to as being located "above" or "below" the second feature, may be in direct contact with the second feature, or in indirect contact with the second feature via an intermediate feature. Moreover, the first feature, when being referred to as being disposed "on", "above" and "over" the second feature, may be disposed right above or obliquely above the second feature, or simply disposed at a level higher than the second feature. The first feature, when being referred to as being disposed "under", "below" and "beneath" the second feature, may be disposed directly below or obliquely below the second feature, or simply disposed at a level lower than the second feature.

It should be noted that, when an element is referred to as being "fixed to" or "disposed on" another element, it may be directly on the other element or may also be present with an intermediate element. When an element is referred to as being "connected" to another element, it may be directly connected to the other element or might be present with an intermediate element at the same time. The terms "vertical", "horizontal", "up", "down", "left", "right" and similar expressions used herein are only for the purpose of illustration, rather than presenting the only ways for implementation.

Unless otherwise defined, all technical and scientific terms used herein have the same meanings as those commonly understood by those skilled in the art of the present disclosure. The terms used in the description of the present disclosure herein are only for the purpose of describing specific embodiments and are not intended to limit the present disclosure. The term 'and/or' used herein includes any and all combinations of one or more related listed items.

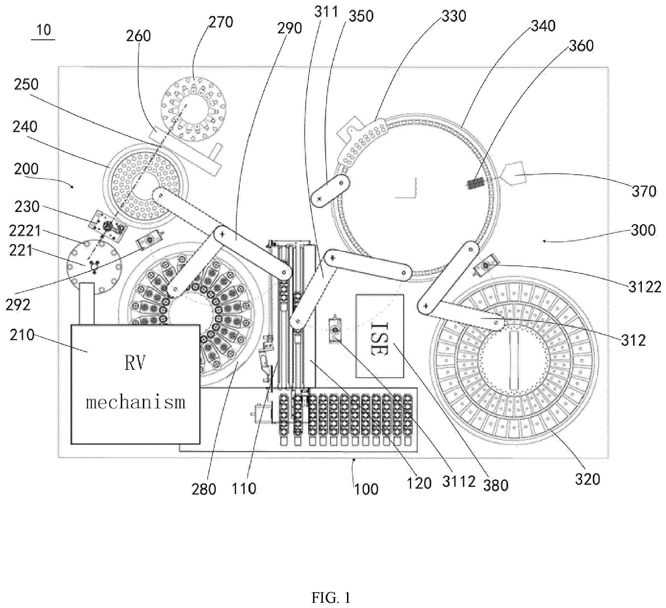

Referring to FIG. 1, an embodiment of the present disclosure provides a sample joint inspection and analysis system 10.

Referring to FIG. 1, the sample joint inspection and analysis system 10 includes a sample processing device 100, a chemiluminescent immunoassay device 200, a biochemical assay device 300, and a machine frame 400. The sample processing device 100, the chemiluminescent immunoassay device 200 and the biochemical assay device 300 are mounted on the machine frame 400. The sample processing device 100 includes an immune sample entering channel 110 and a biochemical sample entering channel 120. The immune sample entering channel 110 is in communication with the chemiluminescent immunoassay device 200, and the biochemical sample entering channel 120 is in communication with the biochemical assay device 300.

The requirements of biochemical reaction and immune reaction on the carrying capacity of a sample adding syringe are different, and such requirement of immune sample addition is much higher than that of biochemical sample addition. Generally, a cleaning method involving cleaning solution would be adopted for the immune sample addition to enhance the cleaning effect. While for the biochemical sample addition, since Na+, K+ and Cl- need to be tested, deionized purified water is generally used for cleaning, and the cleaning solution cannot be used. Therefore, in this embodiment, it is preferred that the immune sample entering channel 110 and the biochemical sample entering channel 1202 are respectively used to add samples respectively, and the two channels will not interfere with each other, thus ensuring the rapid and independent operation of the two analysis devices.

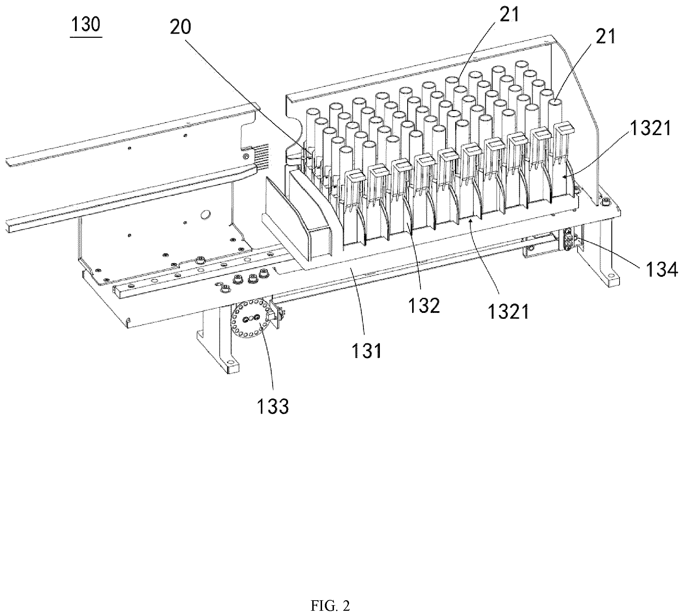

In an embodiment, referring to FIG. 2, the sample processing device 100 further includes a sample entering bin mechanism 130. The sample entering bin mechanism 130 includes a sample entering seat 131, a sample entering rack 132, a first sample entering driving member 133, and a second sample entering driving member 134. The sample entering seat 131 is mounted to the machine frame 400. The sample entering rack 132 is movably connected to the sample entering seat 131. The sample entering rack 132 is provided with a plurality of sample stations 1321 configured to place sample racks 20 in parallel. The first sample entering driving member is connected to the sample entering rack 132 to drive the sample entering rack 132 to move along a first direction. Each of the sample stations 1321 is configured to be aligned with the immune sample entering channel 110 and the biochemical sample entering channel 120 respectively. The second sample entering driving member 134 is mounted to the sample entering seat 131 to apply an action to the sample rack 20 in each sample station 1321, so as to move the sample rack 20 outwards to the immune sample entering channel 110 or the biochemical sample entering channel 120 along a second direction.

In an embodiment, the immune sample entering channel 110 is parallel to the biochemical sample entering channel 120. The first direction is perpendicular to the immune sample entering channel 110 or the biochemical sample entering channel 120. The second direction is parallel to the immune sample entering channel 110 or the biochemical sample entering channel 120. For example, the first direction is the X-axis direction in the horizontal plane, and the second direction is the Y-axis direction in the horizontal plane.

In an embodiment, the sample processing device 100 further includes a barcode scanning mechanism configured to scan a barcode of a blood collection tube 21 containing samples in the immune sample entering channel 110 and the biochemical sample entering channel 120, and identify test items of this blood collection tube 21 to be performed. The barcode scanning mechanism is not shown in the figures.

In an embodiment, the chemiluminescent immunoassay device 200 includes a reaction cup arranging mechanism 210, a reaction cup feeding mechanism 220, a vortex mixer 230, an immune incubator 240, a reaction cup moving mechanism 250, a reading station 260, an immune magnetic separation cleaning mechanism 270330, an immune reagent disk 280, and an immune feeding mechanism 290. The reaction cup arranging mechanism 210 is configured to arrange and order reaction cups 30. The reaction cup feeding mechanism 220 is connected to the reaction cup arranging mechanism 210. The reaction cup feeding mechanism 220 has a plurality of reaction cup 30 stations configured to receive the reaction cups 30. The reaction cup moving mechanism 250 is configured to transfer the reaction cups 30 between the reaction cup feeding mechanism 220, the vortex mixer 230, the immune incubator 240, the reading station 260, and the immune magnetic separation cleaning mechanism 270330. The immune feeding mechanism 290 is configured to obtain an immune reagent and a sample from the immune reagent disk 280 and the immune sample entering channel 110 respectively, and add the immune reagent and the sample into the reaction cup 30 in the immune incubator 240.

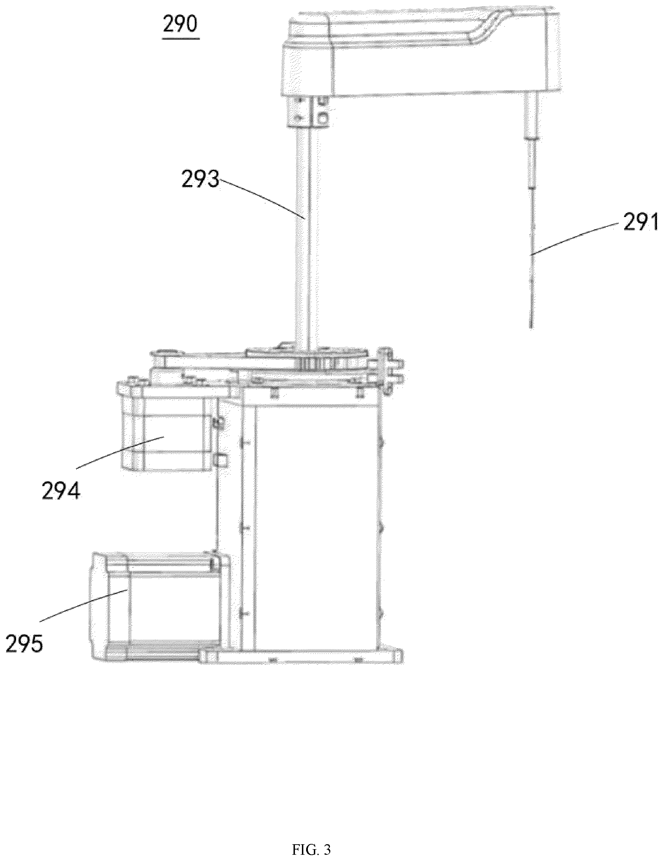

In an embodiment, referring to FIG. 3, the immune feeding mechanism 290 includes an immune sample feeding syringe 291 and an immune syringe cleaning tank 292. Both the immune sample feeding syringe 291 and the immune syringe cleaning tank 292 are provided on the machine frame 400. The immune sample feeding syringe 291 is capable of obtaining the immune reagent and the sample from the immune reagent disk 280 and the immune sample entering channel 110 respectively, and adding the immune reagent and the sample into the reaction cup 30 in the immune incubator 240. The immune syringe cleaning tank 292 is configured to clean the immune sample feeding syringe 291.

In an embodiment, referring to FIG. 3, the immune feeding mechanism 290 further includes an immune sample feeding arm 293, a first immune sample feeding driving member 294, and a second immune sample feeding driving member 295. The immune sample feeding syringe 291 is connected to the immune sample feeding arm 293. The first immune sample feeding driving member 294 is mounted to the machine frame 400 and connected to the immune sample feeding arm 293. The first immune sample feeding driving member 294 is configured to drive the immune sample feeding arm 293 to rotate horizontally, so as to realize transfer of the immune sample feeding syringe 291 between the immune reagent disk 280, the immune sample entering channel 110, the immune incubator 240, and the immune syringe cleaning tank 292.

In an embodiment, the reaction cup arranging mechanism 210, the reaction cup feeding mechanism 220, the vortex mixer 230, the immune incubator 240, the reading station 260, and the immune magnetic separation cleaning mechanism 270330 are sequentially distributed.

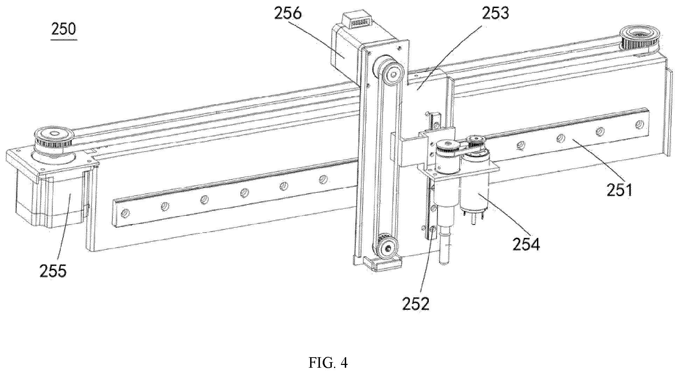

In an embodiment, referring to FIG. 4, the reaction cup moving mechanism 250 includes a first linear guiding member, a second linear guiding member, a reaction cup moving seat 253, a reaction cup gripper 254, a first moving driving member 255, and a second moving driving member 256. The first linear guiding member extends from the reaction cup feeding mechanism 220 to the immune magnetic separation cleaning mechanism 270330. The reaction cup moving seat 253 is slidably connected to the first linear guiding member. The first moving driving member 255 is connected to the reaction cup moving seat 253 to drive the reaction cup moving seat 253 to move along the first linear guiding member. The second linear guiding member is connected to the reaction cup moving seat 253 and extends along a vertical direction. The reaction cup gripper 254 is slidably connected to the second linear guiding member. The second moving driving member 256 is mounted to the reaction cup moving seat 253 and connected to the reaction cup gripper 254. The second moving driving member 256 is configured to drive the reaction cup gripper 254 to move along the second linear guiding member. The first linear guiding member of the reaction cup moving mechanism 250 may be a linear guiding rail. The reaction cup moving mechanism 250 takes up small space and has low cost. It should be understood that, the first linear guiding member can also be replaced by a rotational track or designed as a two-dimensional motion track.

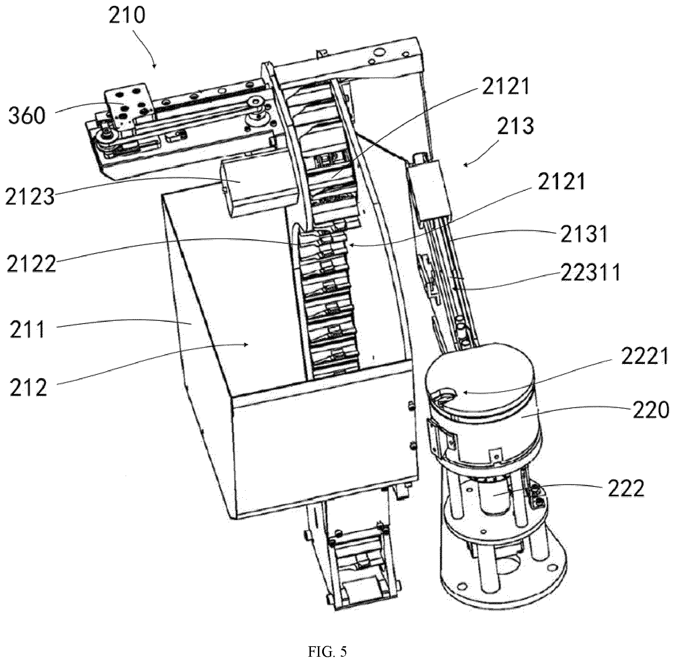

In an embodiment, referring to FIG. 5, the reaction cup arranging mechanism 210 includes a bin 211, a cup collecting assembly 212, and an ordering assembly 213. The cup collecting assembly 212 includes a cup collecting seat 2121, a cup collecting chain 2122, and a cup collecting driving member 2123. A portion of the cup collecting chain 2122 extends into the bin 211 and another portion thereof extends out of the bin 211. A plurality of cup collecting seats 2121 are sequentially connected to the cup collecting chain 2122. The cup collecting seat 2121 is provided with a cup collecting groove 21211 configured to hold one reaction cup 30. The cup collecting driving member 2123 is connected to the cup collecting chain 2122 to drive the cup collecting chain 2122 to move. The ordering assembly 213 includes an ordering track 2131, a transfer pushing member 2132, and a reaction cup transfer driving member 2133. The ordering track 2131 is provided with a chute 21311 configured to receive the reaction cup 30. A height difference is formed between a feeding end and a discharging end of the chute 21311, so that the reaction cup 30 can slide out. The discharging end of the chute 21311 extends to the reaction cup 30 station of the reaction cup feeding mechanism 220. The transfer pushing member 2132 is configured to push the reaction cup 30 in the cup collecting groove 21211 into the feeding end of the chute 21311. The chute 21311 enables the adjustment of the position of the reaction cup 30. After the reaction cup 30 enters the chute 21311, the mouth of the reaction cup 30 is stuck at the groove opening of the chute 21311, and a bottom portion of the reaction cup 30 falls into the chute 21311 under the action of gravity, so that a top portion and the bottom portion of the reaction cup 30 are arranged. The reaction cup 30 automatically slides into the reaction cup 30 station of the reaction cup feeding mechanism 220 under the action of the height difference of the chute 21311. After the reaction cup 30 station of the reaction cup feeding mechanism 220 receives one reaction cup 30, the reaction cup feeding mechanism 220 automatically rotates a distance of one station, so that another empty reaction cup 30 station waits for the next reaction cup 30 to fall in. A time interval of the rotation of the reaction cup 30 station of the reaction cup feeding mechanism 220 is the same as a time interval of the transfer pushing of the transfer pushing member 2132. That is, every time the cup collecting chain 2122 advances, the transfer pushing member 2132 pushes once, and the reaction cup feeding mechanism 220 rotates once, so as to ensure that the reaction cup 30 enter the reaction cup feeding mechanism 220 in sequence. After pushing one reaction cup 30, the transfer pushing member 2132 returns to its initial position, and the cup collecting assembly 212 continues to move upward for one position to align the cup collecting groove 21211 of the next cup collecting seat 2121 with the transfer pushing member 2132, so that scattered reaction cup 30 are orderly arranged in the reaction cup feeding mechanism 220.

After the chemiluminescent immunoassay device 200 completes the analysis, the reaction cup 30 is discarded. Therefore, the reaction cup 30 in the chemiluminescent immunoassay device 200 is a disposable consumable, and the bin 211 needs to be constantly supplemented with new reaction cup 30.

In an embodiment, referring to FIG. 5, the reaction cup feeding mechanism 220 includes a feeding turntable 221 and a rotating driving member 222. The feeding turntable 221 is provided with the plurality of reaction cup 30 stations. The reaction cup 30 stations are distributed at equal intervals along a circumferential direction of the feeding turntable 221. The rotating driving member 222 is connected to the feeding turntable 221 to drive the feeding turntable 221 to rotate.

When the chemiluminescent immunoassay device 200 is in operation, the first linear guiding member, the second linear guiding member, the reaction cup moving seat 253, the reaction cup gripper 254, the first moving driving member 255, and the second moving driving member 256 of the reaction cup moving mechanism 250 cooperate with each other to transfer a single reaction cup 30 from the reaction cup 30 station of the feeding turntable 221 to the immune incubator 240. The immune sample feeding arm 293, the first immune sample feeding driving member 294, and the second immune sample feeding driving member 295 cooperate to drive the immune sample feeding syringe 291 to move. The immune sample feeding syringe 291 firstly obtains the immune reagent from the immune reagent disk 280, and adds the immune reagent to the reaction cup 30 in the immune incubator 240. The immune sample feeding syringe 291 is cleaned in the immune syringe cleaning tank 292, and the cleaned immune sample feeding syringe 291 obtains the sample from the blood collection tube 21 containing samples in the biochemical sample entering channel 120, and adds the sample to the reaction cup 30 in the immune incubator 240. The immune sample feeding syringe 291 is cleaned in the immune syringe cleaning tank 292. After the reaction cup moving mechanism 250 transfers the reaction cup 30 to the vortex mixer 230 for mixing, the reaction cup 30 is transferred to the immune incubator 240 again. After the sample and the immune reagent are incubated and reacted in the immune incubator 240, the reaction cup moving mechanism 250 moves the reaction cup 30 to the magnetic separation cleaning mechanism 330 to complete magnetic separation cleaning. If it is a one-step reaction, after the magnetic separation cleaning is completed, the reaction cup moving mechanism 250 transfers the reaction cup 30 from the magnetic separation cleaning station to the reading station 260, a final optical signal detection is completed through a photoelectric detection of the reading station, and finally the reaction cup 30 in which the reaction is completed is discarded. If it is a two-step reaction, after the magnetic separation cleaning is completed for the first time, the reaction cup moving mechanism 250 moves the reaction cup 30 from the magnetic separation cleaning station back to the immune incubator 240, when the immune sample feeding syringe 291 complete adding the sample and the immune reagent again, the reaction cup 30 is transferred from the immune incubator 240 to the vortex mixer 230 again, and after the mixing is completed on the vortex mixer 230, the reaction cup moving mechanism 250 transfers the reaction cup 30 to the immune incubator 240 again to complete incubation. After the incubation, the reaction cup moving mechanism 250 moves the reaction cup 30 to the magnetic separation cleaning mechanism 330 to complete the magnetic separation cleaning. After the cleaning, the reaction cup moving mechanism 250 reads from the reading station 260 again, and discards the reaction cup after reading, so as to complete the immunoanalysis of the sample. The sample adding sequence of immune reagents and samples may be adjusted as required.

In an embodiment, referring to FIG. 1, the biochemical assay device 300 includes a biochemical feeding mechanism, a biochemical reagent disk 320, a cleaning mechanism 330, a biochemical incubator 340, a stirring mechanism 350, a detection light source 360, an optical detection module 370, and an ISE ion detection module 380, which are provided on the machine frame 400. The biochemical feeding mechanism is mounted to the machine frame 400 to obtain the biochemical reagent and the sample from the biochemical reagent disk 320 and the biochemical sample entering channel 120 respectively, and add the biochemical reagent and the sample to a reaction vessel 40 in the biochemical incubator 340. The stirring mechanism 350 is connected to the machine frame 400 to stir the sample in the reaction vessel 40. The detection light source 360 and the optical detection module 370 are arranged oppositely inside and outside the biochemical incubator 340 to detect an optical signal of the reaction vessel 40. The ISE ion detection module 380 is provided on the machine frame 400 to detect the concentration of one or more ion in the sample. The ISE ion detection module 380 can be configured to detect Na+, K+, and Cl- in the sample. If ion detection is performed on the sample, the biochemical feeding mechanism directly discharges the sample to the ISE ion detection module 380 for ion detection, and does not need to discharge the sample to the reaction vessel 40 of the biochemical incubator 340. The cleaning mechanism 330 is mounted on the machine frame 400 and located above the biochemical incubator 340. The cleaning mechanism 330 is configured to clean the reaction vessel 40 in the biochemical incubator 340.

In an embodiment, the biochemical feeding mechanism includes a first feeding mechanism 311 and a second feeding mechanism 312.

The first feeding mechanism 311 includes a biochemical cleaning tank 3112 and a biochemical sample feeding syringe 3111 that are provided on the machine frame 400. The second feeding mechanism 312 includes a biochemical reagent syringe 3121 and a reagent syringe cleaning tank 3122 that are provided on the machine frame 400. The biochemical sample feeding syringe 3111 is capable of obtaining the sample from the biochemical sample entering channel 120 and adding the sample to the reaction vessel 40 in the biochemical incubator 340. The biochemical syringe cleaning tank is configured to clean the biochemical sample feeding syringe 3111. The biochemical reagent syringe 3121 is capable of obtaining the biochemical reagent from the biochemical reagent disk 320 and adding the biochemical reagent to the reaction vessel 40 in the biochemical incubator 340. The reagent syringe cleaning tank 3122 is configured to clean the biochemical reagent syringe 3121.

In an embodiment, the biochemical sample feeding syringe 3111 and the biochemical reagent syringe 3121 may be used in combination, that is, the biochemical sample feeding syringe 3111 and the biochemical reagent syringe 3121 share one syringe. However, the biochemical reaction is generally relatively fast, and the use of the same syringe head tends to limit the test speed. Therefore, it is preferable to use the biochemical sample feeding syringe 3111 and the biochemical reagent syringe 3121 separately.

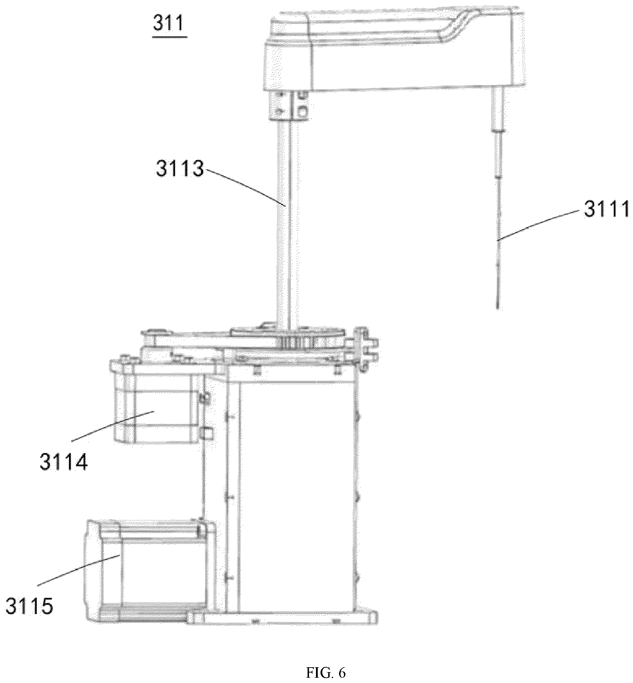

In an embodiment, referring to FIG. 6, the first feeding mechanism 311 includes a biochemical sample feeding arm 3113, a first biochemical sample feeding driving member 31114, and a second biochemical sample feeding driving member 3115. The biochemical sample feeding syringe 3111 is connected to the biochemical sample feeding arm 3113. The first biochemical sample feeding driving member is mounted to the machine frame 400 and connected to the biochemical sample feeding arm 3113. The first biochemical sample feeding driving member 3114 is configured to drive the biochemical sample feeding arm 3113 to rotate horizontally, so as to realize transfer of the biochemical sample feeding syringe 3111 between the immune sample entering channel 110, the biochemical incubator 340, and the biochemical cleaning tank 3112.

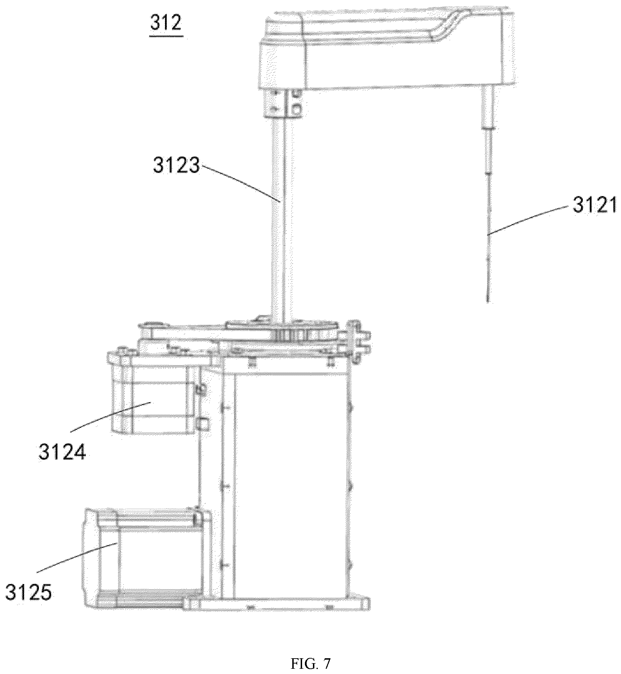

In an embodiment, referring to FIG. 7, the second feeding mechanism 312 includes a reagent feeding arm 3123, a first reagent feeding driving member 3124, and a second reagent feeding driving member 3125. The biochemical reagent syringe 3121 is connected to the reagent feeding arm 3123. The first reagent feeding driving member 3124 is mounted to the machine frame 400 and connected to the reagent feeding arm 3123. The first reagent feeding driving member 3124 is configured to drive the reagent feeding arm 3123 to rotate horizontally, so as to realize transfer of the biochemical reagent syringe 3121 between the biochemical reagent disk 320, the biochemical incubator 340, and the reagent syringe cleaning tank 3122.

The reaction vessel 40 used in the biochemical assay device 300 can be cleaned and recycled to reduce the usage cost. The reaction vessel 40 is generally a reaction cup 30 made of permanent quartz glass. The reaction vessel 40 is fixed on the biochemical incubator 340, and is rotated to move to positions below each component of the biochemical assay device 300 through the biochemical incubator 340, and no additional equipment is required to transfer the reaction vessel 40. The biochemical incubator 340 provides a constant environment temperature for the reaction vessel 40, which is generally set at a constant temperature of 37°C.

When the biochemical assay device 300 is in use, the cleaning mechanism 330 cleans the reaction vessel 40 in the biochemical incubator 340 for several times, and absorbs the liquid in the reaction vessel 40, so that the reaction vessel 40 become clean before being used for a new reaction. The cleaned reaction vessel 40 is advanced in a certain cycle, for example, through the rotation of the biochemical incubator 340. When the cleaned reaction vessel 40 is advanced to a position below the track of the biochemical sample feeding syringe 3111, the biochemical sample feeding syringe 3111 draws the sample from the blood collection tube 21 located on the sample rack 20 in the biochemical sample entering channel 120 and transferred transfers the sample to the reaction vessel 40. During the reaction, it continues to be advanced in the cycle, and rotates to a position below the track of the biochemical reagent syringe 3121, then the biochemical reagent syringe 3121 draws the biochemical reagent from the biochemical reagent bottle in the biochemical reagent disk 320 and transfers the biochemical reagent to the reaction vessel 40. After the biochemical reagent and the sample are added, the reaction vessel 40 is rotated to a position below the stirring mechanism 350 to be stirred and mixed. After fully being stirred and mixed, the reaction vessel 40 is incubated and reacted in the biochemical incubator 340. At the same time, the reaction vessel 40 passes through the optical detection module 370 in each measurement cycle to collect photoelectric data. After the reaction, the reaction vessel 40 is circulated back to a position below the cleaning mechanism 330, and the cleaning mechanism 330 cleans the reaction vessel 40 for several times again, so that the reaction vessel 40 can be recycled.

Inner and outer walls of the biochemical sample feeding syringe 3111 are firstly cleaned in the biochemical cleaning tank 3112. After being cleaned, the biochemical sample feeding syringe 3111 is rotated to the biochemical sample entering channel 120, draws the sample from the blood collection tube 21, and transfers the sample to the corresponding reaction vessel 40 in the biochemical incubator 340. If ISE ion detection of Na⁺, K⁺, Cl⁻ is to be performed, the biochemical sample feeding syringe 3111 directly transfers the sample to the ISE ion detection module 380 for detection without transferring the sample to the reaction vessel 40.

Inner and outer walls of the biochemical reagent syringe 3121 are firstly cleaned in the reagent syringe cleaning tank 3122. After being cleaned, the biochemical reagent syringe 3121 is rotated to the biochemical reagent disk 320. The biochemical reagent disk 320 rotates the reagent kit to be detected to a position below the track of the biochemical reagent syringe 3121. The biochemical reagent syringe 3121 draws the required biochemical reagent and transfers the biochemical reagent to the reaction cup 30 for biochemical reaction. In the case of a two-step reaction, when the biochemical reagent is added for the second time, the reaction vessel 40 will rotate back to the position below the biochemical reagent syringe 3121 again, and the biochemical reagent syringe 312 transfers the biochemical reagent of a second component to the reaction vessel 40 for reaction. Two groups of biochemical reagent syringes 3121 may be provided, and the two groups of biochemical reagent syringe 3121 complete the dispensing of a first component and the second component respectively, so that the whole biochemical reaction process can be accelerated. In terms of cost and space saving and customer demand for test speed, only one group of biochemical reagent syringe 3121 may be used for the dispensing of the first component and the second component. The dispensing sequence of the biochemical reagent and the sample can be adjusted as required.

### Embodiment 1

The present embodiment provides a method for joint detection and analysis of a sample.

The method for joint detection and analysis of the sample of this embodiment uses the above sample joint inspection and analysis system 10. Taking the three-joint-detection of C-reactive protein (CRP), serum amyloid protein A (SAA) and procalcitonin (PCT) in infectious diseases as an example, the operation process of the sample joint inspection and analysis system 10 is described in detail in this embodiment. The PCT is detected through the chemiluminescent immunoassay device 200, while CRP and SAA are biochemical testing items, which are detected through the principle of immunoturbidimetry.

As shown in FIG. 1, the blood of the patient is drawn into the blood collection tube 21, and after being centrifuged to remove the cover, the blood collection tube 21 is placed into the sample rack 20. The sample rack 20 is placed in a sample station 1321 of the sample entering bin mechanism 130. After a sensor (not shown) detects that there is a sample rack 20 in the sample station 1321, the first sample entering driving member drives the sample entering rack 132 to move along the X-axis, and the corresponding bin station can be aligned with the immune sample entering channel 110 or the biochemical sample entering channel 120. The barcode scanning mechanism scans the barcode of the blood collection tube 21 containing the sample in the immune sample entering channel 110 or the biochemical sample entering channel 120, identifies the test items of this blood collection tube 21 to be performed, and obtains the test signals of three items of CRP, SAA, and PCT through the barcode.

The sample joint inspection and analysis system 10 determines which item to be tested first based on the busy level of an immunoluminescent analysis device and the biochemical assay device 300. It is assumed that the PCT is tested first. Then, the second sample entering driving member 134 acts on the sample rack 20 in the sample station 1321 to move the sample rack 20 outward along the Y-axis direction to the immune sample entering channel 110 and transfer the sample rack 20 to a position below the track of the immune injection syringe 291. At the same time, the reaction cup arranging mechanism 210 arranges the reaction cup 30 into an orderly arrangement, and arranges the ordered reaction cup 30 one by one into the reaction cup feeding mechanism 220. The reaction cup 30 transfer mechanism grasps a new reaction cup 30 from the reaction cup feeding mechanism 220 and transfers the reaction cup 30 to one of incubation holes of the immune incubator 240. After being cleaned in the immune cleaning tank, the immune sample feeding syringe 291 draws the corresponding sample from the blood collection tube 21 and transfers the sample to the reaction cup 30 of the immune incubator 240. After the sample required for immune reaction of PCT is drawn, the sample rack 20 returns to the sample entering rack 132, and the sample entering rack 132 aligns with the biochemical sample entering channel 120 again, so that the blood collection tube 21 is transferred to the biochemical sample entering channel 120.

The cleaned immune sample feeding syringe 291 continues to draw immune reagent from the reagent kit in the immune reagent disk 280 and transfer the immune reagent to the reaction cup 30 in which the sample has been added in the immune incubator 240. The reaction cup 30 with immune reagent added is transferred to the vortex mixer 230 by the reaction cup 30 transfer mechanism for vortex mixing. After mixing, the reaction cup 30 is transferred to the immune incubator 240 by the reaction cup 30 transfer mechanism for incubation reaction under constant temperature condition. After the incubation reaction for a predetermined time, the reaction cup 30 is transferred to the magnetic separation cleaning station by the reaction cup 30 transfer mechanism for magnetic separation cleaning. After being cleaned, the reaction cup is transferred to the reading station 260 again by the reaction cup 30 transfer mechanism to perform a detection of a luminescent signal, so as to determine the concentration of PCT.

On the other hand, the blood collection tube 21 has been transferred to the biochemical sample entering channel 120, the biochemical incubator 340 rotates, and after being cleaned several times by the cleaning mechanism 330, at least two clean reaction vessels 40 are obtained. The two cups 40 rotate and advance to the position below the track of the biochemical sample feeding syringe 3111 in sequence, and the amounts of the sample required for the two items of CRP and SAA item are respectively added through the biochemical sample feeding syringe 3111. Then, the two reaction vessels 40 continue to advance and reach the position below the track of the biochemical reagent syringe 3121 in sequence. The cleaned biochemical reagent syringe 3121 draws biochemical reagents required for the reaction from the CRP reagent bottle and the SAA reagent bottle in the biochemical reagent disk 320 respectively and add them to the two reaction vessels 40 respectively. The two reaction vessels 40 continue to rotate and advance to the position below the stirring mechanism 350 to complete the stirring and mixing of the sample and the biochemical reagent. After mixing, the incubation reaction is continued in the biochemical incubator. At the same time, the photoelectric signals are collected and detected by the optical detection module 370, and the detections of the two items of CRP and SAA are performed by the turbidimetry method. After the detections of the three items, the detection results are transmitted and fed back to the patient in the form of a report sheet or a Lis information system, so as to achieve the multi-item joint detection with one tube of blood, one operation mode and one unified result.

The above-mentioned sample joint inspection and analysis system 10 is miniaturized in structure and rapid in detection, which can realize multi-item joint detection based on biochemical reaction principle and chemiluminescent detection principle, and take the testing advantages of both the biochemical and immune reactions, and the two test modes will not interfere with each other, which can meet the needs of multi-item joint detection with high throughput and high sensitivity in various medical and testing institutions or units. At the same time, it also provides patients with multiple options. The biochemical assay and immunoassay of the above-mentioned sample joint inspection and analysis system 10 are performed simultaneously, realizing high-throughput biochemical-immune multiple-item joint detection.

The technical features of the embodiments above may be combined arbitrarily. To make the description concise, not all possible combinations of the technical features in the above embodiments are described. However, as long as there are no contradictions in the combinations of these technical features, all of the combinations should be considered to be within the scope of the specification.

The embodiments above only represent several implementation modes of the present disclosure, and the description thereof is relatively specific and detailed, but it should not be construed as limiting the scope of the patent. It should be noted that for those skilled in the art, various modifications and improvements may be made without departing from the concept of the present disclosure, and all these modifications and improvements belong to the protection scope of the present disclosure. Therefore, the scope of protection of the patent disclosure should be subject to the appended claims.

## Claims

1. A sample joint inspection and analysis system, comprising:
a machine frame; and
a sample processing device, a chemiluminescent immunoassay device, and a biochemical assay device that are provided on the machine frame;
wherein the sample processing device comprises an immune sample entering channel and a biochemical sample entering channel, the immune sample entering channel is in communication with the chemiluminescent immunoassay device, and the biochemical sample entering channel is in communication with the biochemical assay device.

2. The sample joint inspection and analysis system according to claim 1, wherein the sample processing device further comprises a sample entering bin mechanism, the sample entering bin mechanism comprises a sample entering seat, a sample entering rack, a first sample entering driving member, and a second sample entering driving member, the sample entering seat is mounted to the machine frame, the sample entering rack is movably connected to the sample entering seat, the sample entering rack is provided with a plurality of sample stations configured to place sample racks in parallel, the first sample entering driving member is connected to the sample entering rack to drive the sample entering rack to move along a first direction, each of the sample stations is configured to be aligned with the immune sample entering channel and the biochemical sample entering channel respectively, and the second sample entering driving member is mounted to the sample entering seat to apply an action to the sample rack in each sample station, so as to move the sample rack outwards to the immune sample entering channel or the biochemical sample entering channel along a second direction.

3. The sample joint inspection and analysis system according to claim 1, wherein the chemiluminescent immunoassay device comprises a reaction cup arranging mechanism, a reaction cup feeding mechanism , a vortex mixer , an immune incubator, a reaction cup moving mechanism, a reading station, an immune magnetic separation cleaning mechanism, an immune reagent disk, and an immune feeding mechanism, the reaction cup arranging mechanism is configured to arrange and order reaction cups, the reaction cup feeding mechanism is connected to the reaction cup arranging mechanism, the reaction cup feeding mechanism has a plurality of reaction cup stations configured to receive the reaction cups, the reaction cup moving mechanism is configured to transfer the reaction cup between the reaction cup feeding mechanism, the vortex mixer, the immune incubator, the reading station, and the immune magnetic separation cleaning mechanism, and the immune feeding mechanism is configured to obtain an immune reagent and a sample from the immune reagent disk and the immune sample entering channel respectively, and add the immune reagent and the sample into the reaction cup in the immune incubator.

4. The sample joint inspection and analysis system according to claim 3, wherein the immune feeding mechanism comprises an immune sample feeding syringe and an immune syringe cleaning tank, both the immune sample feeding syringe and the immune syringe cleaning tank are provided on the machine frame, the immune sample feeding syringe is capable of obtaining the immune reagent and the sample from the immune reagent disk and the immune sample entering channel respectively, and adding the immune reagent and the sample into the reaction cup in the immune incubator, and the immune syringe cleaning tank is configured to clean the immune sample feeding syringe; and/or
the reaction cup arranging mechanism, the reaction cup feeding mechanism, the vortex mixer, the immune incubator, the reading station, and the immune magnetic separation cleaning mechanism are sequentially distributed; and/or
the reaction cup arranging mechanism comprises a bin, a cup collecting assembly, and an ordering assembly, the cup collecting assembly comprises cup collecting seats, a cup collecting chain, and a cup collecting driving member, a portion of the cup collecting chain extends into the bin and another portion thereof extends out of the bin , a plurality of the cup collecting seats are sequentially connected to the cup collecting chain, the cup collecting seat is provided with a cup collecting groove configured to hold one reaction cup, the cup collecting driving member is connected to the cup collecting chain to drive the cup collecting chain to move, the ordering assembly comprises an ordering track, a transfer pushing member, and a reaction cup transfer driving member, the ordering track is provided with a chute configured to receive the reaction cup, a height difference is formed between a feeding end and a discharging end of the chute to enable the reaction cup to slide out, the discharging end of the chute extends to the reaction cup station of the reaction cup feeding mechanism, and the transfer pushing member is configured to push the reaction cup in the cup collecting groove into the feeding end of the chute.

5. The sample joint inspection and analysis system according to claim 4, wherein the immune feeding mechanism further comprises an immune sample feeding arm, a first immune sample feeding driving member, and a second immune sample feeding driving member, the immune sample feeding syringe is connected to the immune sample feeding arm, the first immune sample feeding driving member is mounted to the machine frame and connected to the immune sample feeding arm, and the first immune sample feeding driving member is configured to drive the immune sample feeding arm to rotate horizontally, so as to transfer the immune sample feeding syringe between the immune reagent disk, the immune sample entering channel, the immune incubator, and the immune syringe cleaning tank.

6. The sample joint inspection and analysis system according to claim 4, wherein the reaction cup moving mechanism comprises a first linear guiding member, a second linear guiding member, a reaction cup moving seat, a reaction cup gripper, a first moving driving member, and a second moving driving member, the first linear guiding member extends from the reaction cup feeding mechanism to the immune magnetic separation cleaning mechanism, the reaction cup moving seat is slidably connected to the first linear guiding member, the first moving driving member is connected to the reaction cup moving seat to drive the reaction cup moving seat to move along the first linear guiding member, the second linear guiding member is connected to the reaction cup moving seat and extends along a vertical direction, the reaction cup gripper is slidably connected to the second linear guiding member, the second moving driving member is mounted to the reaction cup moving seat and connected to the reaction cup gripper, and the second moving driving member is configured to drive the reaction cup gripper to move along the second linear guiding member.

7. The sample joint inspection and analysis system according to claim 4, wherein the reaction cup feeding mechanism comprises a feeding turntable and a rotating driving member, the feeding turntable is provided with a plurality of reaction cup stations distributed at equal intervals along a circumferential direction of the feeding turntable, and the rotating driving member is connected to the feeding turntable to drive the feeding turntable to rotate.

8. The sample joint inspection and analysis system according to anyone of claims 1 to 7, wherein the biochemical assay device comprises a biochemical feeding mechanism, a biochemical reagent disk, a cleaning mechanism, a biochemical incubator, a stirring mechanism, a detection light source, an optical detection module, and an ISE ion detection module, the biochemical feeding mechanism is mounted to the machine frame to obtain a biochemical reagent and a sample from the biochemical reagent disk and the biochemical sample entering channel respectively, and add the biochemical reagent and the sample to a reaction vessel in the biochemical incubator, the stirring mechanism is connected to the machine frame to stir the sample in the reaction vessel, the detection light source and the optical detection module are arranged oppositely inside and outside the biochemical incubator to detect an optical signal of the reaction vessel, the ISE ion detection module is provided on the machine frame to detect the concentration of one or more ion in the sample, and the cleaning mechanism is configured to clean the reaction vessel in the biochemical incubator.

9. The sample joint inspection and analysis system according to claim 8, wherein the biochemical feeding mechanism comprises a biochemical cleaning tank, a biochemical sample feeding syringe, a biochemical reagent syringe, and a reagent syringe cleaning tank that are provided on the machine frame, the biochemical sample feeding syringe is capable of obtaining the sample from the biochemical sample entering channel and adding the sample to the reaction vessel in the biochemical incubator, the biochemical syringe cleaning tank is configured to clean the biochemical sample feeding syringe, the biochemical reagent syringe is capable of obtaining the biochemical reagent from the biochemical reagent disk and adding the biochemical reagent to the reaction vessel in the biochemical incubator, and the reagent syringe cleaning tank is configured to clean the biochemical reagent syringe.

10. The sample joint inspection and analysis system according to claim 9, wherein the biochemical feeding mechanism further comprises a biochemical sample feeding arm, a first biochemical sample feeding driving member, and a second biochemical sample feeding driving member, the biochemical sample feeding syringe is connected to the biochemical sample feeding arm, the first biochemical sample feeding driving member is mounted to the machine frame and connected to the biochemical sample feeding arm, and the first biochemical sample feeding driving member is configured to drive the biochemical sample feeding arm to rotate horizontally, so as to transfer the biochemical sample feeding syringe between the immune sample entering channel, the biochemical incubator, and the biochemical cleaning tank; and/or
the biochemical feeding mechanism further comprises a reagent feeding arm , a first reagent feeding driving member, and a second reagent feeding driving member, the biochemical reagent syringe is connected to the reagent feeding arm, the first reagent feeding driving member is mounted to the machine frame and connected to the reagent feeding arm, and the first reagent feeding driving member is configured to drive the reagent feeding arm to rotate horizontally, so as to transfer the biochemical reagent syringe between the biochemical reagent disk, the biochemical incubator, and the reagent syringe cleaning tank.
